(19) Europäisches Patentamt
European Patent Office
Office européen des brevets

(11) EP 1 367 109 B1

(12) **EUROPEAN PATENT SPECIFICATION**

(45) Date of publication and mention of the grant of the patent:
**11.05.2005 Bulletin 2005/19**

(51) Int Cl.[7]: **C09J 7/02**, C09J 11/06, A61L 24/04

(21) Application number: **03012062.0**

(22) Date of filing: **28.05.2003**

(54) **Pressure sensitive adhesive sheet**

Druck-abhängige Haftmittel

Compositions adhésives par pression

(84) Designated Contracting States:
**AT BE BG CH CY CZ DE DK EE ES FI FR GB GR HU IE IT LI LU MC NL PT RO SE SI SK TR**

(30) Priority: **29.05.2002 JP 2002154999**

(43) Date of publication of application:
**03.12.2003 Bulletin 2003/49**

(73) Proprietor: **NITTO DENKO CORPORATION
Osaka (JP)**

(72) Inventors:
- **Murakami, Yoshihide, c/o Nitto Denko Corporation Ibaraki-shi, Osaka (JP)**
- **Okada, Katsuhiro, c/o Nitto Denko Corporation Ibaraki-shi, Osaka (JP)**

(74) Representative: **Grünecker, Kinkeldey, Stockmair & Schwanhäusser Anwaltssozietät
Maximilianstrasse 58
80538 München (DE)**

(56) References cited:
**EP-A- 0 624 635**
**EP-A- 1 184 039**
**US-A- 3 949 742**
**US-A- 5 153 040**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 367 109 B1

## Description

This invention relates to pressure sensitive adhesive sheets, for example, it relates to a pressure sensitive adhesive sheet for medical use and sanitary materials.

It is required that a pressure sensitive adhesive tape for medical use and sanitary materials can inhibit invasion of water, bacteria, viruses and the like from the outside and also has a flexibility which can follow curved surface and movement of the skin. Thus, as the support for such a pressure sensitive adhesive tape, a thin layer elastomer film having low elastic modulus similar to the skin is generally used. Also, excellent moisture permeability is required for a pressure sensitive adhesive tape for medical use and sanitary materials, e.g., a dressing material and the like, for effecting evaporation of moisture by perspiration from the skin into the outside. When its moisture permeability is poor, moisture generated from the skin retains between the skin and an adhesive layer, so that the pressure sensitive adhesive sheet tends to lose its fixing ability by causing reduction of adhesive strength of its adhesive layer and skin disorders tend to occur by inducing a maceration phenomenon of the skin due to the water retained on the skin surface. Accordingly, block polymers having a polyether backbone as a hydrophilic unit, or the like have been used as the support for dressing materials and the like.

As the adhesive to be used in the adhesive layer, an adhesive comprising a (meth)acrylic acid ester polymer having excellent adhesiveness and moisture permeability and low chemical stimulus to the skin is generally used. However, since the adhesive comprising a (meth)acrylic acid ester polymer has strong adhesive power, it sometimes causes pain when a pressure sensitive adhesive tape is peeled off from the skin and damages keratin layer and epidermis of the skin. Particularly, when pressure sensitive adhesive tapes are repeatedly applied to the same region, skin damages which accompany bleeding occur in some cases, thus causing a serious problem.

With the purpose of reducing such physical stimuli to the skin, an adhesive wherein a (meth)acrylic acid ester polymer is allowed to contain a large amount of a liquid component that is compatible with this polymer and made into a gel by carrying out a crosslinking treatment has been proposed. For example, such an adhesive is disclosed in JP-A-6-23029 or JP-A-6-319793, which can soften and disperse the stress to the skin surface at the time of peeling, while keeping the high adhesiveness of the (meth)acrylic acid ester polymer. Accordingly, since the physical stimuli to the skin are low and peeling of keratin and the like does not occur, this is being applied to transdermal absorption type tape preparations and surgical tapes for medical treatment use.

However, when a pressure sensitive adhesive tape is formed by laminating the adhesive disclosed above on the conventional elastomer film comprising a polyether block polymer, a liquid component in the adhesive is transferred into the elastomer film to cause swelling and deformation of the film. Accordingly, the processing suitability such as stamping processability of the film is extremely deteriorated. In this connection, some elastomer films other than the polyether block polymer can control the swelling deformation at a low level, but it is poor in moisture permeability and is not suited for pressure sensitive adhesive tapes for medical use which are frequently applied to the skin and the like.

EP-A-1184039 relates to an adhesive composition for application to skin, comprising an acrylic copolymer obtained from a monomer mixture comprising a (meth)acrylic acid alkyl ester monomer, an alkoxy group-containing ethylenically unsaturated monomer and a carboxy group-containing ethylenically unsaturated monomer, and a carboxylic acid ester, which is liquid or paste at room temperature.

EP-A-624635 discloses a medical pressure-sensitive adhesive comprising an acrylic polymer and a liquid or pasty component which is compatible with the acrylic polymer. The liquid or pasty component comprises at least one ester of a monobasic cid or a polybasic acid and a branded alcohol, and an ester of an unsaturated fatty acid or a branched acid and a tetrahydric or lower hydric alcohol. 50 to 80% by weight of the acrylic polymer is insolubilized by crosslinking.

US-A-153040 relates to a wound dressing comprising a film wherein at least one edge of the film is an edge portion which is separate by perforations from the remainder of the film; a pressure-sensitive adhesive coated on at least a portion of one surface of the film; and a liner which is adhered to the edge portion with sufficient tenacity to result in separation along the perforations of the edge portion from the remainder of the film before the liner separates from the edge portion and which liner is releasably adhered to the remainder of the film.

It was the object of the present invention to provide a pressure sensitive adhesive sheet which has a moderate adhesive strength, can control physical stimuli to the adherend at the time of peeling and also has good processing suitability such that the support does not show swelling deformation by transfer of a liquid component from the adhesive layer.

This object is achieved by a pressure sensitive adhesive sheet comprising

(i) an adhesive layer formed from a resin composition containing an acrylic acid ester polymer, a carboxylic acid ester having 16 or more carbon atoms which is compatible with said acrylic acid ester polymer and is liquid or paste at ordinary temperature, and a crosslinking component, and

(ii) a film supporting said adhesive layer, comprising a polyether urethane resin obtained by allowing a polyol

component to react with a polyisocyanate component, wherein the polyol component contains at least one kind of polyether polyol having a polyoxyalkylene backbone whose repeating unit is represented by the following formula (1):

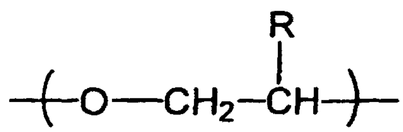

(1)

(wherein R represents H or an alkyl group).

According to the invention, liquid component-absorbing ability of a support can be markedly reduced and a pressure sensitive adhesive sheet having such an excellent processing suitability and showing no swelling deformation can be produced, by preparing the pressure sensitive adhesive sheet through the combination of a support comprising a resin composition of specified components with an adhesive of a specified composition.

The pressure sensitive adhesive sheet of the invention has excellent moisture permeability. Thus, when it is used, e.g., for the human body and the like as tapes for medical use or tapes for sanitary material use, it does not cause stuffiness and does not cause unpleasant feeling during its application. In addition, since the pressure sensitive adhesive sheet of the invention has a low elastic modulus equivalent to the skin and also has flexibility which can follow curved surface and movement of the skin, its peeling and the like do not occur even when a curvature movement is made after applying it as a plaster to a finger or the like. In this connection, when the term "film" is used in the invention, it means a general idea of including a sheet, and the term "sheet" of including a film.

In this specification, the term "molecular weight" means the weight average molecular weight.

The pressure sensitive adhesive sheet of the invention has an adhesive layer on the support. The support is a film comprising a polyether urethane resin, and the polyol component constituting the polyether urethane resin contains at least one kind of polyether polyol having a polyoxyalkylene backbone whose repeating unit is represented by the following formula (1) (to be referred to as "polyether polyol having a polyoxyalkylene backbone" hereinafter). The polyether urethane resin is obtained by allowing this polyol component to react with a polyisocyanate component.

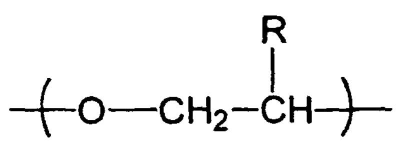

(1)

(In the formula, R represents H or an alkyl group.)

The polyether polyol having a polyoxyalkylene backbone is obtained by polymerizing at least one of alkylene oxides (e.g., ethylene oxide, propylene oxide, butylene oxide and the like) represented by the aforementioned formula (1). Polyether polyol having a polyoxyalkylene backbone having a molecular weight of from 500 to 3,000 is suitably used. In the formula (1), the alkyl group in the meaning of R preferably has 1 or 2 carbon atoms.

Regarding the polyol component constituting a polyether urethane resin, a polyol having a polyoxyalkylene backbone but having other structure than the polyoxyalkylene backbone whose repeating unit is represented by the formula (1) may be copolymerized with a polyether polyol having a polyoxyalkylene backbone. As an example of the other polyoxyalkylene backbone, a polyoxytetramethylene backbone can be cited, and a copolymerized product thereof can be used. When the polyol component is a copolymer, it may be either a random copolymer or a block copolymer.

According to the invention, as the polyol component, the polyether polyol having a polyoxyalkylene backbone can be used by partially mixing with other polyol, e.g., polyoxytetramethylene glycol or butanediol, heptanediol, hexanediol, cyclohexane dimethanol or the like.

The polyether urethane resin formed using the polyol component containing a polyether polyol having a polyoxyalkylene backbone has low absorption for an adhesive obtained from a specified carboxylic acid ester which will be described later. Though the reason for this is not completely clear, it is considered that this is due to reduction of affinity of the polyether urethane resin with a carboxylic acid ester having high hydrophobicity, e.g., a carboxylic acid ester having 16 or more carbon atoms, when a polyether structure having high polarity is present in the polyether urethane resin.

As the polyisocyanate component which constitutes the aforementioned polyether urethane resin, a conventionally known material can be used. For example, aromatic, aliphatic and alicyclic diisocyanates and dimers, trimers and the like of these diisocyanates can be cited. Examples of the aromatic, aliphatic and alicyclic diisocyanates include tolylene diisocyanate, diphenylmethane diisocyanate, hexamethylene diisocyanate, xylylene diisocyanate, hydrogenated xylylene diisocyanate, isophorone diisocyanate, hydrogenated diphenylmethane diisocyanate, 1,5-naphthylene diisocyanate, 1,3-phenylene diisocyanate, 1,4-phenylene diisocyanate, butane-1,4-diisocyanate, 2,2,4-trimethylhexamethylene diisocyanate, 2,4,4-trimethylhexamethylene diisocyanate, cyclohexane-1,4-diisocyanate, dicyclohexylmethane-4,4-diisocyanate, 1,3-bis(isocyanatemethyl)cyclohexane, methylcyclohexane diisocyanate, m-tetramethylxylylene diisocyanate and the like. Also, their dimers and trimers and polyphenylmethane polyisocyanate can be used. As the trimers, isocyanurate types, burette types, allophanate types and the like can be exemplified, and they can be used optionally. These polyisocyanates may be used alone or as a mixture of two or more.

According to the invention, a chain elongation agent can be further used. As the chain elongation agent, a conventionally known material can be used, and its examples include ethylene glycol, propylene glycol, butanediol and the like diols and ethylenediamine, tolylenediamine, isophoronediamine and the like diamines.

According to the invention, as occasion demands, additives generally used in films, such as an ultraviolet ray absorbent, an antioxidant, a filler, a pigment, a coloring agent, a flame retarder, an antistatic agent and the like, can be added generally. These additives are used in the amounts generally used according to their kinds.

The polyether urethane resin can be polymerized by, e.g., using a one-shot method or a pre-polymer method. Also, even in the case of bulk polymerization which does not use a solvent, for the purpose of reducing viscosity, the polymerization may be carried out in a solution.

The bulk polymerization is illustratively described in the following. A polyol is put into a reaction vessel and its urethanation is effected by adding a polyisocyanate while stirring and controlling the temperature to 50 to 80°C. After further carrying out the reaction by adding a chain elongation agent, a mass-form polyether urethane resin can be obtained by transferring the reaction product on a tray and completing the reaction by keeping it at 100 to 150°C for 4 hours or more.

A film comprising a polyether polyurethane resin is formed by pulverizing and pelletizing the mass-form polyether urethane resin, and then melting this resin pellet and extruding it into a sheet form using T-die extruder or inflation die extruder. In this connection, the film extruded into a film form is generally wound up. Alternatively, a film comprising a polyether polyurethane is formed by a calender processing in which a polyether urethane resin is made into a sheet by rolling and extending it between two heated rolls, and the film is wound up as occasion demands. Also, a film comprising a polyether urethane resin may be formed by dissolving the resin pellet in N,N-dimethylformamide (DMF) or the like solvent, coating this solution, e.g., on a polyester film or the like release liner using a bar coater or the like, drying it to remove the solvent.

In the case of a pressure sensitive adhesive sheet of the present invention to be used for medical use or sanitary material use, it is desirable that thickness of the film comprising a polyether urethane resin is within a range of from 10 to 150 μm. When thickness of the film is less than 10 μm, it becomes difficult to handle in applying or peeling it to or from the skin so that the workability is reduced to a practically impossible level. On the other hand, when thickness of the film is thicker than 150 μm, sufficient moisture permeability cannot be obtained so that it becomes unsuitable for a pressure sensitive adhesive sheet which is based on the assumption that it is applied to the skin. When the pressure sensitive adhesive sheet is used for dressing purpose, it is particularly desirable that thickness of the film is within the range of from 20 to 60 μm.

In this connection, the support can be made into a multiple layer structure, and for example, it may be made into a laminate of polyether urethane films.

The pressure sensitive adhesive sheet of the invention has an adhesive layer on the support, illustratively, it has an adhesive layer on a film comprising the aforementioned polyether urethane resin. The adhesive layer is obtained by mixing an acrylic acid ester polymer, a carboxylic acid ester compatible with this acrylic acid ester polymer and a crosslinking agent and applying a crosslinking treatment thereto. The carboxylic acid ester has 16 or more carbon atoms and is liquid or paste at ordinary temperature (at 23°C).

The acrylic acid ester polymer is a substance having a (meth)acrylic acid ester as the main component and, as occasion demands, copolymerized with a copolymerizable monomer. As the (meth)acrylic acid ester, those in which the number of carbon atoms of an alkyl group is 2 or more, preferably the number of carbon atoms is 2 or more and 18 or less, can be exemplified. For example, ethyl ester, propyl ester, butyl ester, pentyl ester, hexyl ester, octyl ester, nonyl ester, decyl ester, dodecyl ester and the like of (meth)acrylic acid can be cited. According to the invention, one or two or more of these (meth)acrylic acid esters are used. Also, the alkyl ester chain thereof may be either straight chain or branched chain.

Examples of the monomer copolymerizable with the (meth)acrylic acid ester include monomers containing carboxyl group, such as (meth)acrylic acid, itaconic acid, maleic acid and the like; monomers containing hydroxyl group, such as 2-hydroxyethyl (meth)acrylate, 2-hydroxypropyl (meth)acrylate and the like; and monomers containing an

alkoxy group, such as methoxyethyl (meth)acrylate, ethoxyethyl (meth)acrylate, butoxyethyl (meth)acrylate, methoxy (meth)acrylate polyethylene glycol, ethoxy (meth)acrylate diethylene glycol and the like, styrene or styrene derivatives, vinyl acetate, N-vinyl-2-pyrrolidone and the like. As occasion demands, one or two or more of these monomers can be used by copolymerizing with the (meth)acrylic acid ester.

It is desirable that the acrylic acid ester polymer has a glass transition temperature of 260°K or less. By setting the glass transition temperature of the acrylic acid ester polymer to 260°K or less, sufficient expression of the skin adhesiveness becomes possible so that it becomes desirable as an adhesive layer of a pressure sensitive adhesive sheet for medical use or sanitary material use.

It is desirable that the acrylic acid ester polymer is controlled to have a molecular weight of 1,000,000 or less, preferably about 500,000 to about 900,000. When the molecular weight is too large, skin adhesiveness somtimes becomes insufficient due to the too strong cohesiveness within the adhesive layer.

The acrylic acid ester polymer can be obtained by known polymerization method such as a solution polymerization, emulsion polymerization, suspension polymerization or the like. It can also be obtained by carrying out radical polymerization using a peroxide system compound, azo system compound or the like radical polymerization initiator.

The carboxylic acid ester to be mixed with the acrylic acid ester polymer has good compatibility with the acrylic acid ester polymer. Also, the carboxylic acid ester to be used in the invention shows a liquid or paste state at ordinary temperature, and solid states (wax and the like) are excluded therefrom. This is because the adhesive property is decreased when the adhesive layer is formed by mixing a solid state carboxylic acid ester.

According to the invention, a gel-form adhesive layer can be obtained by mixing an acrylic acid ester polymer with a carboxylic acid ester and a crosslinking agent and forming a crosslinked substance in at least a portion thereof. The adhesive layer obtained in this manner can reduce elastic modulus in the infinitesimal deformation region and can exert good adhesive property to the skin by improving adhesiveness (wettability) of the adhesive layer surface to irregular surface of the skin. What is more, since it can lighten and disperse stress applied to the skin surface when the pressure sensitive adhesive sheet is peeled, it exerts such effects that physical stimuli are hardly added to the skin surface at the time of its peeling, peeling and the like of keratin on the skin surface hardly occur too, and damage on the skin is also extremely small.

Examples of the carboxylic acid ester to be used preferably in the invention include esters which use monohydric alcohols, such as ethyl myristate, isopropyl myristate, isopropyl palmitate, butyl stearate, isopropyl isostearate, hexyl laurate, cetyl lactate, myristyl lactate, diethyl phthalate, dioctyl phthalate, octyldodecyl myristate, octyldodecyl oleate, hexyldecyl dimethyloctanoate, cetyl 2-ethylhexanoate, isocetyl 2-ethylhexanoate, stearyl 2-ethylhexanoate, dioctyl succinate and the like, and esters which use polyhydric (dihydric or more) alcohols, such as dicaprylic acid propylene glycol, dicapric acid propylene glycol, diisostearic acid propylene glycol, glyceryl monocaprylate, glyceryl tricaprylate, glyceryl tri-2-ethylhexanoate, glyceryl tricaprate, glyceryl trilaurate, glyceryl triisistearate, glyceryl trioleate, trimethylolpropane tri-2-ethylhexanoate and the like.

It is necessary that the number of carbon atoms of the carboxylic acid ester to be used herein is 16 or more. That is, when the number of carbon atoms of the carboxylic acid ester is 15 or less, the processing suitability is reduced due to swelling deformation of the film caused by absorption of a large amount of the liquid component by the film. In this connection, when an adhesive layer formed by mixing a carboxylic acid ester having 16 or more carbon atoms is laminated on a urethane resin film formed as the support by using a polyol having the repeating unit represented by the aforementioned formula (1), the liquid component is hardly absorbed by the film so that deformation of the film becomes minimum.

According to the invention, at least one of the aforementioned carboxylic acid esters is dissolved in the acrylic acid ester polymer. Though its formulating amount is not particularly limited, for example, it is desirable that the carboxylic acid esters is contained within a range of from 30 to 100 parts by mass based on 100 parts by mass of the acrylic acid ester polymer (i.e., within a range of from 30 to 100 parts by weight based on 100 parts by weight of the acrylic acid ester polymer).

According to the invention, it is necessary that a crosslinked substance is formed in a portion of the acrylic acid ester polymer in which the aforementioned carboxylic acid ester is dissolved. A crosslinking treatment is carried out in order to form the crosslinked substance, for example, a chemical crosslinking treatment may be carried out using an organic peroxide, an isocyanate compound, an organic metal salt, a metal chelate, an epoxy compound or the like, or physical crosslinking treatment may be carried out using an ionizing radiation.

The crosslinking treatment is carried out so that the gel fraction ratio (solvent-insoluble fraction ratio) becomes from 30 to 80 wt%, preferably from 35 to 70 wt%. A gel fraction ratio of less than 30 wt% would cause insufficient cohesive power within the adhesive layer, which results in pollution of the skin with a remaining adhesive when the sheet is peeled off from the skin. On the other hand, a gel fraction ratio of more than 80 wt% would cause a lowered skin adhesiveness, which results in partial peeling at the end of the sheet or detachment or drop of the sheet during application of the sheet to the skin.

As occasion demands, the resin composition (adhesive) which forms the adhesive layer can be mixed with

various additives such as plasticizers (e.g., glycerol, polyethylene glycol and the like), aqueous or water absorbing resins (e.g., polyacrylic acid, polyvinyl pyrrolidone and the like), tackifiers (e.g., rosin-based, terpene-based, and petroleum-based tackifiers), and various softening agents, fillers, pigments and the like. Particularly, when a substance having an unsaturated bond is used as the carboxylic acid ester, there is a possibility that desired characteristics are not exerted due to generation of changes in physical properties caused by oxidation deterioration by oxygen in the air, so that it is desirable to formulate a conventionally known antioxidant in the resin composition (adhesive).

It is desirable to set thickness of the adhesive layer within the range of from 10 to 80 μm. When thickness of the adhesive layer is less than 10 μm, sufficient adhesive property is not exerted in some cases, and when it exceeds 80 μm, moisture permeability at the level required for the pressure sensitive adhesive sheet for skin sticking use cannot be obtained in some cases.

As has been described in the foregoing, a pressure sensitive adhesive sheet having high moisture permeability can be obtained by selecting materials of the support and adhesive as specified in the pressure sensitive adhesive sheet of the invention. When a pressure sensitive adhesive sheet is applied to the human skin surface, it is necessary that the moisture permeability when it is stored at 40°C for 24 hours under an atmosphere of 30% RH is 600 $g/m^2 \cdot 24$ h·40°C·30 RH or more, though it slightly varies depending on the individual difference and region to be applied. When a pressure sensitive adhesive sheet having a moisture permeability of less than this is applied to the skin for 1 week or more, continuous stuffiness is generated which becomes the cause of skin stimulation. Accordingly, it is desirable that moisture permeability of the pressure sensitive adhesive sheet for use in the application to the skin is set within the range of from 800 $g/m^2 \cdot 24$ h·40°C·30 RH to 2,400 $g/m^2 \cdot 24$ h·40°C·30 RH.

In this case, the moisture permeability of a film is the amount of water vapor which permeates per 1 $m^2$ of the film under a predetermined condition, which is a value converted into per 1 $m^2$ of the decreased amount of water when a predetermined amount of water is put into a container having a predetermined opening diameter, and the container opening is covered with a film which is then allowed to stand under conditions of a temperature of 40°C and a relative humidity of 30% RH for 24 hours. It can be said that stuffiness of the film is small when the moisture permeability is high.

According to the invention, tapes and sheets for medical use such as plasters can be formed using this pressure sensitive adhesive sheet. For example, a plaster can be formed by cutting the pressure sensitive adhesive sheet into an appropriate size, or it can be formed into tapes or sheets for medical use such as a cladding material for covering wounded regions, a protector after surgical operation, a material for covering the inserting part of catheters, gauze and the like, and products for medical use, such as fixing tapes, tool maintaining tapes and the like, can be formed by combining the pressure sensitive adhesive sheet with other base material and the like.

The following describes the invention in detail using examples. In this connection, the term "part(s)" as used in the following examples means part(s) by mass.

(Inventive Example 1)

As polyols, 200 g (0.2 mol) of polytetramethylene glycol (PTMG) having a molecular weight of 1,000 and 65 g (0.03 mol) of polyethylene glycol (PEG) having a molecular weight of 2,000 were put into a reaction vessel equipped with a condenser, a heating device, a thermometer and a stirring device and mixed. While stirring this at a temperature of 70°C, 190 g (0.76 mol) of methylenediphenyl diisocyanate (MDI) heated to 50°C was added thereto as a polyisocyanate, followed by stirring for 5 minutes. As a chain elongation agent, 45 g (0.50 mol) of 1,4-butanediol (BD) heated at 50°C was added thereto, followed by stirring for 5 minutes to carry out the reaction. Thereafter, the reaction product was transferred to a tray and put into a hot air dryer to carry out aging at 140°C for 5 hours, thereby obtaining a mass-form polyether urethane resin. The resulting mass-form polyether urethane resin was pulverized and dissolved in N, N-dimethylformamide (DMF) to prepare a solution of 30% in concentration. This solution was cast on a release-treated polyester film (38 μm in thickness) to an after-drying thickness of 30 μm using a bar coater and dried at 160°C for 5 minutes to obtain a polyether urethane film as the support.

An acrylic acid ester polymer was obtained by copolymerizing isononyl acrylate (NA), methoxyethyl acrylate (MEA) and acrylic acid (AA). In this case, the formulation ratio of the acrylic acid ester polymer was NA:MEA:AA = 65 g:30 g:5 g. A solution for adhesive layer was prepared by mixing 100 g of the resulting acrylic acid ester polymer with 60 g of isopropyl myristate (IPM) as a carboxylic acid ester component and 0.25 g of a trifunctional isocyanate compound as a crosslinking agent component in toluene. This solution for adhesive layer was coated on the silicone-treated surface of a silicone-treated polyethylene laminate paper (release liner) to an after-drying thickness of 30 μm and then dried to form an adhesive layer.

The resulting adhesive layer was pasted on the prepared polyurethane film surface and then stored under an atmosphere of 60°C for 3 days to complete crosslinking reaction of the adhesive layer, thereby preparing a pressure sensitive adhesive sheet for dressing material use. In this connection, the number of carbon atoms constituting isopropyl myristate is 16.

(Inventive Example 2)

A pressure sensitive adhesive sheet for dressing material use was prepared in the same manner as in Inventive Example 1, except that the kind and the amount of the carboxylic acid ester component of Inventive Example 1 were changed to 80 g of glyceryl tricaprylate (GTC). In this connection, the number of carbon atoms constituting glyceryl tricaprylate is 27.

(Inventive Example 3)

As polyols, 250 g (0.13 mol) of a polyoxypropylene polyoxyethylene copolymer (PPPEG) (molecular weight 2,000, weight ratio of polyoxyethylene 75%) and 20 g (0.01 mol) of polytetramethylene glycol (PTMG) having a molecular weight of 2,000 were put into a reaction vessel equipped with a condenser, a heating device, a thermometer and a stirring device and mixed. While stirring this at a temperature of 70°C, 180 g (0.72 mol) of methylenediphenyl diisocyanate (MDI) heated to 50°C was added thereto as a polyisocyanate, followed by stirring for 5 minutes. As a chain elongation agent, 50 g (0.56 mol) of 1,4-butanediol (BD) heated at 50°C was added thereto, followed by stirring for 5 minutes to carry out the reaction. Thereafter, the reaction product was transferred to a tray and put into a hot air dryer to carry out aging at 140°C for 5 hours, thereby obtaining a mass-form polyether urethane resin.

A polyether urethane film was obtained in the same manner as in Inventive Example 1, except that the resulting mass-form polyether urethane resin was used. Next, a pressure sensitive adhesive sheet for dressing material use was prepared in the same manner as in Inventive Example 1, except that the resulting polyurethane film was used.

(Inventive Example 4)

A pressure sensitive adhesive sheet for dressing material use was prepared in the same manner as in Inventive Example 3, except that the kind and the amount of the carboxylic acid ester component for the preparation of adhesive layer in Inventive Example 3 were changed to 50 g of propylene glycol dicaprylate (PGDC). In this connection, the number of carbon atoms constituting propylene glycol dicaprylate is 19.

(Inventive Example 5)

A pressure sensitive adhesive sheet for film dressing use was prepared in the same manner as in Inventive Example 3, except that the kind and the amount of the carboxylic acid ester component for the preparation of the adhesive layer in Inventive Example 3 were changed to 100 g of glyceryl tri-2-ethylhexanoate (GTEH). In this connection, the number of carbon atoms constituting glyceryl tri-2-ethylhexanoate is 19.

(Comparative Example 1)

As a polyol, 290 g (0.15 mol) of polytetramethylene glycol (PTMG) having a molecular weight of 2,000 was put into a reaction vessel equipped with a condenser, a heating device, a thermometer and a stirring device. While stirring at 70°C, 165 g (0.66 mol) of methylenediphenyl diisocyanate (MDI) heated to 50°C was added thereto as a polyisocyanate, followed by stirring for 5 minutes. As a chain elongation agent, 45 g (0.50 mol) of 1,4-butanediol (BD) heated at 50°C was added thereto, followed by stirring for 5 minutes to carry out the reaction. Thereafter, the reaction product was transferred to a tray and put into a hot air dryer to carry out aging at 140°C for 5 hours, thereby obtaining a mass-form polyether urethane resin.

A polyether urethane film was obtained in the same manner as in Inventive Example 1, except that the resulting mass-form polyether urethane resin was used. Next, a pressure sensitive adhesive sheet for dressing material use was prepared in the same manner as in Inventive Example 1, except that the resulting polyether urethane film was used.

(Comparative Example 2)

A pressure sensitive adhesive sheet for dressing use was prepared in the same manner as in Comparative Example 1, except that the kind and the amount of the carboxylic acid ester component for the preparation of adhesive layer in Comparative Example 1 were changed to 100 g of glyceryl tricaprylate (GTC). In this connection, the number of carbon atoms constituting propylene glycol dicaprylate is 19.

(Comparative Example 3)

A pressure sensitive adhesive sheet for dressing material use was prepared in the same manner as in Inventive Example 1, except that, in Inventive Example 1, a film (30 μm in thickness) formed from a polyamide resin ("PEBAX 3533" mfd. by Atochem) was used as the support instead of the polyether urethane film, and the kind and the amount of the carboxylic acid ester component for the preparation of adhesive layer were changed to 60 g of glyceryl tricaprylate (GTC). In this connection, it was identified based on the results of $^1H$ and $^{13}C$ nuclear magnetic resonance analyses that composition of the polyamide resin "PEBAX 3533" is a polyether amide resulting from the reaction of E-caprolactone (CL), adipic acid (AD) and polytetramethylene glycol (PTMG).

(Comparative Example 4)

A pressure sensitive adhesive sheet for dressing material use was prepared in the same manner as in Inventive Example 1, except that, in Inventive Example 1, a film (30 μm in thickness) formed from a polyester resin ("FLECMER" mfd. by The Nippon Synthetic Chemical Industry Co., Ltd.) was used as the support instead of the polyether urethane film, and the kind and the amount of the carboxylic acid ester component for the preparation of adhesive layer were changed to 60 g of glyceryl tri-2-ethylhexanoate (GTEH). In this connection, it was identified based on the results of $^1H$ and $^{13}C$ nuclear magnetic resonance analyses that composition of the polyester resin "FLECMER" is a polyether ester resulting from the reaction of butylene terephthalate (BT), polytetramethylene glycol (PTMG) and butanediol (BD).

(Comparative Example 5)

A pressure sensitive adhesive sheet for dressing material use was prepared in the same manner as in Inventive Example 1, except that, in Inventive Example 1, a film (30 μm in thickness) formed from a polyurethane resin ("Elastollan C85A11" mfd. by BASF Polyurethane Elastomer) was used as the support instead of the polyether urethane film, and the kind and the amount of the carboxylic acid ester component for the preparation of adhesive layer were changed to 80 g of glyceryl tricaprylate (GTC). In this connection, it was identified based on the results of $^1H$ and $^{13}C$ nuclear magnetic resonance analyses that composition of the polyurethane resin "Elastollan C85A11" is-a polyester urethane resulting from the reaction of methylenediphenyl diisocyanate (MDI), adipic acid (AD), butanediol (BD) and hexanediol (HD).

(Comparative Example 6)

A pressure sensitive adhesive sheet for dressing material use was prepared in the same manner as in Inventive Example 1, except that the kind and the amount of the carboxylic acid ester component for the preparation of adhesive layer in Inventive Example 1 were changed to 80 g of butyl caprylate (BC). In this connection, the number of carbon atoms constituting butyl caprylate is 12.

(Comparative Example 7)

A pressure sensitive adhesive sheet for dressing material use was prepared in the same manner as in Inventive Example 1, except that the kind and the amount of the carboxylic acid ester component for the preparation of adhesive layer in Inventive Example 1 were changed to 80 g of butyl caproate (BCp). In this connection, the number of carbon atoms constituting butyl caproate is 10.

(Evaluation Tests)

Evaluation tests of the following absorption ratio and deformation ratio of films were carried out on the films as supports obtained in Inventive Examples 1 to 5 and Comparative Examples 1 to 7, and evaluation tests of processing suitability and moisture permeability shown below were carried out on the pressure sensitive adhesive sheets. The results are shown in Table 1.

(1) Evaluation of carboxylic acid ester absorbing property to support film

Each support film was cut into a square of 50 mm x 50 mm in size, and its weight and the length of a side of the square were measured. This sample film was soaked in a solution of the carboxylic acid ester to be measured and stored at a temperature of 50°C for 3 days. Thereafter, the sample film was taken out from the carboxylic acid ester solution, held between paper wipers and pressed to wipe up excess carboxylic acid ester solution on the surface, and then weight of the sample film was measured. Also, the length of a side of the square of the sample film was measured. Based on the following equations, liquid component-absorbing ratio of the support film and deformation ratio of the

support film were calculated.

Absorption ratio (%) = [{(film weight after soaking) - (film weight before soaking)}/(support weight before soaking)] x 100

Deformation ratio (%) = [{(side length of film after soaking) - (side length of film before soaking)}/(side length of support before soaking)] x 100

(2) Evaluation of processing suitability

Using each pressure sensitive adhesive sheet for dressing material use after completion of the crosslinking reaction by heating, its closely contacted side with the release liner was observed with the naked eye, and lifting condition of the adhesive layer was evaluated based on the following criteria. Also, each laminate of the pressure sensitive adhesive sheet and release liner was stamped using a Thomson blade, the stamping-treated laminate was observed with the naked eye, and its suitability for the processing was evaluated based on the following criteria. Evaluation criteria:

O ··· A case in which lifting from release liner was not observed on the pressure sensitive adhesive sheet, and a pressure sensitive adhesive sheet having the same size of the stamping blade was obtained by the stamping
X ··· A case in which lifting from release liner was partially observed on the pressure sensitive adhesive sheet, and a pressure sensitive adhesive sheet having a difference in size with the stamping blade was obtained due to partial difference formed on the cutting face by the stamping

(3) Evaluation of moisture permeability

A 20 ml portion of purified water was put into a glass container having an opening diameter of 40 mm, the glass container opening was covered with a sample, and the glass container was sealed by wrapping outer periphery of the opening with a pressure sensitive adhesive tape. After measuring weight of this glass container, this was stored under conditions of a temperature of 40°C and a relative humidity of 30% RH for 24 hours. By measuring weight of the glass container, the amount of permeated water vapor was calculated from the difference in weight before and after the storage. Amount of permeated moisture (moisture permeability) per 1 $m^2$ of the sample was calculated from this value.

Table 1

| | | Support | Carboxylic acid ester | | | Absorption ratio (%) | Deformation ratio (%) | Processing suitability | Moisture permeability (g/m$^2$) |
|---|---|---|---|---|---|---|---|---|---|
| | | | Kind | Carbon atoms | Compounding amt. (parts) | | | | |
| Inv. Ex. | 1 | Polyether urethane | Isopropyl myristate | 16 | 60 | 8 | 3 | O | 1260 |
| | 2 | Polyether urethane | Glyceryl tricaprylate | 27 | 80 | 12 | 2 | O | 1200 |
| | 3 | Polyether urethane | Isopropyl myristate | 16 | 80 | 9 | 2 | O | 1320 |
| | 4 | Polyether urethane | Propylene glycol dicaprylate | 19 | 50 | 12 | 3 | O | 1400 |
| | 5 | Polyether urethane | Glyceryl tri-2-ethylhexanoate | 27 | 100 | 10 | 3 | O | 1250 |
| Comp. Ex. | 1 | Polyether urethane | Isopropyl myristate | 16 | 60 | 20 | 10 | X | 880 |
| | 2 | Polyether urethane | Glyceryl tricaprylate | 27 | 100 | 21 | 8 | X | 820 |
| | 3 | Polyether amide | Glyceryl tricaprylate | 27 | 60 | 52 | 15 | X | 1280 |
| | 4 | Polyether ester | Glyceryl tri-2-ethylhexanoate | 27 | 60 | 39 | 22 | X | 990 |
| | 5 | Polyester urethane | Glyceryl tricaprylate | 27 | 80 | 11 | 2 | O | 580 |
| | 6 | Polyether urethane | Butyl caprylate | 12 | 80 | 16 | 6 | X | 1280 |
| | 7 | Polyether urethane | Butyl caproate | 10 | 80 | 24 | 9 | X | 1300 |

As is evident from Table 1, it was found that the polyether urethane films of Inventive Examples 1 to 5 of the invention have low absorption ratio into films and deformation ratio of the films is also small. Also, it was found that the

pressure sensitive adhesive sheets of Inventive Examples 1 to 5 have good processing suitability, also have high moisture permeability and do not cause stuffiness and the like even when they are adhered for a prolonged period of time. It was found also that these pressure sensitive adhesive sheets are excellent in transparency and also excellent in skin-following ability.

It was found that the films of Comparative Examples 1 to 4 and 6 and 7 have high liquid component-absorbing ratio and large deformation ratio of films. It was found that the pressure sensitive adhesive sheets having these films as their supports are poor in processing suitability. Though the film of Comparative Example 5 showed low liquid component-absorbing ratio, small film deformation ratio and excellent processing suitability, the pressure sensitive adhesive sheet having this film as the support was poor in moisture permeability so that it could not be applied to pressure sensitive adhesive sheets to be adhered to the skin and the like.

According to the invention, it is possible to provide a pressure sensitive adhesive sheet having excellent transparency, which can inhibit invasion of water, bacteria, viruses and the like from the outside, and also has high moisture permeability, can evaporate moisture due to perspiration from the skin into the outside and does not cause reduction of adhesive strength of the adhesive layer. Also, it can provide a pressure sensitive adhesive sheet which has a moderate adhesive strength, can control physical stimuli to the adherend at the time of peeling and does not generate skin damage. According to the invention, it is possible to provide a pressure sensitive adhesive sheet having such a good processing suitability that the support does not generate swelling deformation by transfer of a liquid component from the adhesive layer.

## Claims

**1.** A pressure sensitive adhesive sheet comprising:

an adhesive layer formed from a resin composition comprising an acrylic acid ester polymer, a carboxylic acid ester having 16 or more carbon atoms which is compatible with said acrylic acid ester polymer and is liquid or paste at ordinary temperature, and a crosslinking component, and

a film supporting said adhesive layer, comprising a polyether urethane resin obtained by allowing a polyol component to react with a polyisocyanate component, wherein the polyol component contains at least one kind of polyether polyol having a polyoxyalkylene backbone whose repeating unit is represented by the following formula (1):

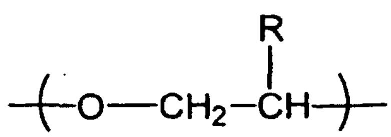

(1)

(wherein R represents H or an alkyl group).

**2.** The pressure sensitive adhesive sheet according to claim 1, wherein the polyether polyol has a weight average molecular weight of from 500 to 3,000.

**3.** The pressure sensitive adhesive sheet according to claim 1, wherein R in the formula (1) is an alkyl group having a number of carbon atoms of from 1 to 2.

**4.** The pressure sensitive adhesive sheet according to claim 1, wherein the acrylic acid ester polymer has a glass transition temperature of 260°K or less.

**5.** The pressure sensitive adhesive sheet according to claim 1, wherein the carboxylic acid ester is from 30 to 100 parts by mass based on 100 parts by mass of the acrylic acid ester polymer.

**6.** The pressure sensitive adhesive sheet according to claim 1, wherein the film has a thickness of from 10 μm to 150 μm.

**7.** The pressure sensitive adhesive sheet according to claim 1, wherein the adhesive layer has a thickness of from

10 µm to 80 µm.

8. The pressure sensitive adhesive sheet according to claim 1, which has a moisture permeability when stored at 40°C for 24 hours under an atmosphere of 30% RH is 600 g/$m^2$·24 h·40°C·30 RH or more.

9. The pressure sensitive adhesive sheet according to claim 8, wherein the moisture permeability is from 800 g/$m^2$·24 h·40°C·30 RH to 2,400 g/$m^2$·24 h·40°C·30 RH.

10. A pressure sensitive adhesive sheet for medical use or sanitary use, which comprises the pressure sensitive adhesive sheet according to claim 1.

**Patentansprüche**

1. Druckempfindliche Klebefolie, umfassend:

   eine Klebstoffschicht, die aus einer ein Acrylsäureesterpolymer enthaltenden Harzzusammensetzung, einem Carbonsäureester mit 16 oder mehr Kohlenstoffatomen, der mit dem Acrylsäureesterpolymer verträglich ist und bei gewöhnlicher Temperatur flüssig oder pastenförmig ist, und einer Vemetzungskomponente gebildet ist, und
   einem diese Klebstoffschicht tragenden Film, umfassend ein Polyetherurethanharz, das durch Umsetzen einer Polyolkomponente mit einer Polyisocyanatkomponente erhalten wird, worin die Polyolkomponente wenigstens eine Art von Polyetherpolyol mit einem Polyoxyalkylengrundgerüst enthält, dessen sich wiederholende Einheit durch die folgende Formel (1) wiedergegeben wird:

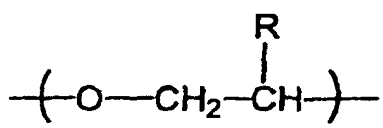

(1)

   (worin R H oder eine Alkylgruppe bedeutet).

2. Druckempfindliche Klebefolie gemäß Anspruch 1, worin das Polyetherpolyol ein Molekulargewicht-Gewichtsmittel von 500 bis 3000 hat.

3. Druckempfindliche Klebefolie gemäß Anspruch 1, worin R in der Formel (1) eine Alkylgruppe mit 1 bis 2 Kohlenstoffatomen ist.

4. Druckempfindliche Klebefolie gemäß Anspruch 1, worin das Acrylsäureesterpolymer eine Glasübergangstemperatur von 260°K oder weniger hat.

5. Druckempfindliche Klebefolie gemäß Anspruch 1, worin der Carbonsäureester 30 bis 100 Masseteile, bezogen auf 100 Masseteile des Acrylsäureesterpolymers, ausmacht.

6. Druckempfindliche Klebefolie gemäß Anspruch 1, worin der Film eine Dicke von 10 µm bis 150 µm hat.

7. Druckempfindliche Klebefolie gemäß Anspruch 1, worin die Klebstoffschicht eine Dicke von 10 µm bis 80 µm hat.

8. Druckempfindliche Klebefolie gemäß Anspruch 1, die, wenn sie 24 Stunden bei 40°C unter einer Atmosphäre von 30 % RH (relative Feuchte) gelagert wird, eine Feuchtigkeitsdurchlässigkeit von 600 g/$m^2$ • 24 h • 40°C • 30 RH oder mehr hat.

9. Druckempfindliche Klebefolie gemäß Anspruch 8, worin die Feuchtigkeitsdurchlässigkeit 800 g/$m^2$ • 24 h • 40°C • 30 RH bis 2400 g/$m^2$ • 24 h • 40°C • 30 RH beträgt.

10. Druckempfindliche Klebefolie für medizinische Verwendung oder sanitäre Verwendung, welche die druckempfindliche Klebefolie gemäß Anspruch 1 umfasst.

**Revendications**

1. Feuille adhésive sensible à la pression, comprenant :

   une couche adhésive formée à partir d'une composition de résine comprenant un polymère d'ester d'acide acrylique, un ester d'acide carboxylique ayant 16 atomes de carbone ou plus qui est compatible avec ledit polymère d'ester d'acide acrylique et est un liquide ou une pâte à la température ordinaire, et un composant de réticulation, et
   un film supportant ladite couche adhésive, comprenant une résine polyéther uréthane obtenue en laissant un composant polyol réagir avec un composant polyisocyanate, le composant polyol contenant au moins une sorte de polyéther polyol ayant un squelette polyoxyalkylène dont le motif répétitif est représenté par la formule (1) suivante :

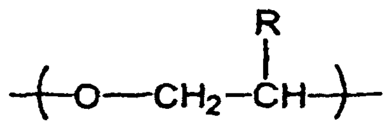

(1)

   (dans laquelle R représente H ou un groupe alkyle).

2. Feuille adhésive sensible à la pression selon la revendication 1, dans laquelle le polyéther polyol a une masse moléculaire moyenne en poids de 500 à 3.000.

3. Feuille adhésive sensible à la pression selon la revendication 1, dans laquelle R, dans la formule (1), est un groupe alkyle ayant un nombre d'atomes de carbone de 1 à 2.

4. Feuille adhésive sensible à la pression selon la revendication 1, dans laquelle le polymère d'ester d'acide acrylique a une température de transition vitreuse de 260°K ou moins.

5. Feuille adhésive sensible à la pression selon la revendication 1, dans laquelle l'ester d'acide carboxylique est de 30 à 100 parties en poids pour 100 parties en poids du polymère d'ester d'acide acrylique.

6. Feuille adhésive sensible à la pression selon la revendication 1, dans laquelle le film a une épaisseur de 10 µm à 150 µm.

7. Feuille adhésive sensible à la pression selon la revendication 1, dans laquelle la couche adhésive a une épaisseur de 10 µm à 80 µm.

8. Feuille adhésive sensible à la pression selon la revendication 1, qui a une perméabilité à l'humidité, lorsqu'on la stocke à 40°C pendant 24 heures dans une atmosphère à 30 % d'HR, qui est de 600 $g/m^2$ . 24 h . 40°C . 30 d'HR, ou plus.

9. Feuille adhésive sensible à la pression selon la revendication 8, dans laquelle la perméabilité à l'humidité est de 800 $g/m^2$ . 24 h . 40°C. 30 d'HR à 2.400 $g/m^2$ . 24 h . 40°C . 30 d'HR.

10. Feuille adhésive sensible à la pression destinée à un usage médical ou sanitaire, qui comprend la feuille adhésive sensible à la pression selon la revendication 1.